# EUROPEAN PATENT APPLICATION

(11) **EP 2 474 322 A1**
(43) Date of publication of application: **11.07.2012**
(21) Application number: 12153349.1
(22) Date of filing: 10.04.2008
(51) Int. Cl.: A61K 39/395, C07K 16/10, C07K 16/06, A61P 31/14

(54) **Products for prophylaxis and/or treatment of viral diseases and method of making and using same**

(30) Priority: 12.04.2007 US 91137307 P
(62) Divisional of application: 08826091.4
(71) Applicant: Omrix Biopharmaceuticals Ltd., 76106 Rehovot (IL)
(72) Inventor: Laub, Orgad, Tel Aviv (IL); Nur, Israel, Moshav Timorim (IL); Orr, Nadav, Mazkeret Batia (IL)
(74) Representative: Carpmaels & Ransford

(57) **Abstract**

The invention relates to an immunoglobulin composition suitable for prophylaxis and/or treatment of a viral mediated disease, disorder or condition and to methods of its preparation. In one embodiment, the invention provides a potent intravenous immunoglobulin composition useful for the treatment or prevention of a West Nile virus mediated disease, disorder or condition.

## Description

### Field of the invention

The invention relates to prophylaxis and/or treatment of a viral mediated disease, disorder or condition such as West Nile Fever.

### Background of the invention

West Nile Virus (WNV), a mosquito transmitted virus of the Flaviviridae family (Scherret, Poidinger et al. 2001) is found in both tropical and temperate regions. It infects birds, humans, horses, dogs, cats, bats, chipmunks, skunks, squirrels, and domestic rabbits. The clinical manifestations of WNV infection in humans range from asymptomatic seroconversion to severe meningoencephalitis with symptoms including cognitive dysfunction, muscle weakness, flaccid paralysis and death (Hayes and Gubler 2006). Depressed immunity, age and genetic factors were correlated with greater risk to contract the neurological disease due to WNV infection (Yakub, Lillibridge et al. 2005). Currently there is no effective therapy or vaccine for patients severely infected with WNV but supportive care.

The West Nile Virus was first isolated from a feverish adult woman in the West Nile District of Uganda in 1937. The ecology was characterized in Egypt in the 1950s. The virus became recognized as a cause of severe human meningoencephalitis in elderly patients during an outbreak in Israel in 1957. The virus and disease further appeared in new geographical areas. Until 1999 West Nile Virus was found in Africa, the Middle East, parts of Asia, southern Europe and Australia. It then suddenly emerged in New York, and rapidly spread throughout the United States causing considerable acute morbidity and mortality (Steele, Linn et al. 2000; Nash, Mostashari et al. 2001). West Nile Virus has been described in Africa, Europe, the Middle East, west and central Asia, Oceania (subtype Kunjin), and North America.

In 2000, there were 21 reported WNV cases in the USA suffering from clinical presentations such as encephalitis, febrile disease, and meningitis. The disease caused 2 deaths. During 2003 the outbreak peaked in the United States with a total of 9,862 cases involving severe meningitis or encephalitis or fever and 264 deaths. During 2004-2007, there were an average of about 3,350 cases in the USA and 130 deaths annually (CDC-USA 2008).

The presence of moderate titers of anti-WNV antibodies in intravenous immunoglobulin (IVIG) produced from Israeli plasma and treatment of two immunocompromised patients suffering from severe WNV infection was reported in two articles (Shimoni et al., 2001 and Hamdan et al., 2002). A standard dose of 0.4 g/kg IVIG (Omr-IgG-am™ 5%, OMRIX Biopharmaceuticals, IL) was administered to two immunosuppressed Israeli patients with serologically confirmed WNV encephalitis. The two patients suffered from severe disease and were unconsciousness, or in deep coma when treated with IVIG. The two articles reported possible beneficial effects of the WNV-antibody positive IVIG that reversed the course of the disease. However, not all batches of Israeli IVIG are expected to be equally effective for treatment of a West Nile Virus related disease, disorder or condition. For example, Hamdan et al., stated that: "The dramatic response of this patient led to the discovery that certain batches of immunoglobulins from Israeli donors had high IgG titers against WNV (11; E. Mendelson personal communication)". Nevertheless, during the years 2005-2007, IVIG produced from Israeli plasma was anecdotally used in the USA for treatment of a number of cases of severe WNV infection without clear effectiveness (Shimoni et al., 2006, and Shimoni et al., patent application).

It was reported that there was a significant variability in WNV antibody levels found in IVIG produced in the USA after 2000, when the virus was already endemic in some regions of the continental USA (Planitzer, Modrof et al. 2007). Thus, regular IVIG is far from serving as a reliable source of immunoglobulin preparation to challenge WNV outbreaks that are widely anticipated by the health community.

US patent 4,617,379 to Dobkin et al. describes a method for the preparation of cytomegalovirus (CMV) hyperimmune serum globulin. Human CMV is a medically important beta herpes virus carried by a majority of individuals, where it is a leading cause of opportunistic and congenital disease (Slobedman B. and ES Mocarski, 1999). Specific antibody for CMV was reported to be found in at least 50% of the population (Kondoet al., 1996). Thus, in order to produce a hyperimmune serum globulin that has a specific titer by far higher than the average population, Dobkin teaches preparation of serum globulin by screening plasma donations from nonvaccinated donors and selecting and pooling only plasma having a sufficiently high titer of antibody. For instance, Dobkin plasma donations which showed a titer of antibody equal to or greater than about 1:60,000 was pooled and used in the Dobkin method for the preparation of the immune serum globulin. The description of Dopkin is silent on the isolation and pooling of all plasma from donors positive for antibody specific to a pathogenic virus strain from a population in a geographic area endemic to the virus in which the percentage of seropositives to said virus in the population is equal or less than 25%.

Currently, there is no effective universal and consistent treatment or anti-viral therapy specific to WNV. Those severely affected require intensive supportive therapy, often involving prolonged hospitalization, administration of large volumes of intravenous fluids, airway management, respiratory support, prevention or treatment of secondary infections (pneumonia, urinary tract, etc.), and good nursing care. Consequently, there is a long felt need for the rapid development of an effective drug to treat WNV infections and to serve as an immediate countermeasure to the expected epidemic and spread of WNV.

### Summary of the invention

In one aspect, the invention provides a method for the preparation of an immunoglobulin composition comprising a high level of neutralizing antibody specific for a pathogenic virus causing a new emerging disease, disorder or condition in a target geographic area. The method comprises screening blood or blood fractions of a plurality of donors from a population of a geographic area endemic to the virus, and isolating and pooling all blood or blood fractions of donors that are positive for antibody specific to the pathogenic virus strain. According to the invention, in such a population the percentage of seropositive donors to the virus is not more than 25%.

In one embodiment of the invention, the percentage of seropositive donors to the virus is in the range of 1-15%. In another embodiment of the invention, the percentage of seropositive donors to the virus is in the range of 8-13%.

In another embodiment of the invention, the screening of blood or blood fractions is carried out with a pathogenic virus strain isolated from or nearby the target geographic area.

In yet another embodiment of the invention, the method further comprises processing the pooled blood or blood fractions to an intravenous immunoglobulin composition.

In a further embodiment of the invention, the method further comprises the step of aliquoting the immunoglobulin composition in a low-volume unit dosage form.

In another embodiment of the invention, the virus is West Nile Virus (WNV), the geographic area endemic to the virus is Israel and/or neighboring territories and the target geographic area is in the United States.

Another aspect of the invention relates to a method for the preparation of an immunoglobulin composition comprising a high level of anti-West Nile Virus (WNV) neutralizing antibody for treating or preventing a disease, disorder or condition caused by WNV in a target geographic area. The method comprises screening blood or blood fractions of a plurality of donors from a population of a geographic area endemic to the virus, and isolating and pooling all blood or blood fractions of donors that are positive for antibody against WNV. According to the invention, in such a population the percentage of seropositives donors to the virus is not more than 25%.

In one embodiment of the invention, the percentage of seropositive donors to the virus is in the range of 1-15%. In another embodiment of the invention, the percentage of seropositive donors to the virus is in the range of 8-13%.

In an embodiment of the invention, the blood or blood fractions are positives for WNV isolated from the target geographic area.

In another embodiment of the invention, the method further comprises processing the pooled blood or blood fractions to an intravenous immunoglobulin composition.

In still another embodiment of the invention, the method further comprises the step of aliquoting the immunoglobulin composition in a low-volume unit dosage form.

In yet another embodiment of the invention, the geographic area endemic to the virus is Israel and/or neighboring territories and the target geographic area is in the United States.

One object of the present invention is to provide a potent immunoglobulin composition comprising high levels of neutralizing antibodies specific for a pathogenic virus causing a new emerging disease, disorder or condition in a target geographic area, obtained by the above specified methods.

Another object of the invention is to provide a potent immunoglobulin composition comprising high levels of antibodies specific for WNV of a target geographic area, wherein the composition comprises immunoglobulin from pooled blood or blood fractions of donors that are all positives for antibody against WNV, wherein such donors are from a population of a geographical area endemic to WNV, and wherein the composition has a high level of antibody against the WNV of the target geographical area that is more than 5 times, 10 times, 11 times, or about 20 times higher than the level of anti-WNV antibody of immunoglobulin from a regular pool of blood or blood fractions of donors of the geographic area endemic to the virus.

In another aspect, the invention relates to a vial or pre-filled syringe containing a low-volume unit dosage of the composition according to the invention.

In yet another aspect of the invention, the invention provides a pharmaceutical composition for the treatment or prevention of a WNV associated disease, disorder or condition in a target geographical area comprising a pharmaceutically acceptable carrier and an immunoglobulin composition according to the invention.

In a further aspect of the invention, the invention provides a method for treating or preventing a WNV associated disease, disorder or condition comprising administering to a subject in need a therapeutically effective amount of an immunoglobulin composition according to the invention.

Still another object of the present invention is to provide a method for treating or preventing a WNV associated disease, disorder or condition which would require administration of an unreachable excessive amount and/or volume of IVIG from a regular pool of donors of the geographic area endemic to the virus, the method comprising administering to a subj ect in need a therapeutically effective amount of an immunoglobulin composition according to the invention.

The invention also provides an efficient method for screening and selecting blood or blood fractions having a high level of neutralizing antibody specific for a pathogenic virus causing a disease, disorder or condition from numerous blood or blood fractions samples, using the steps of: screening blood or blood fraction samples obtained from donors of a population from a geographic area endemic to the virus using an ELISA assay; and selecting blood or blood fractions of donors that are positive for antibody specific for the pathogenic virus, wherein in the population the percentage of seropositives donors to the virus is not more than 25%.

In one embodiment of the invention, the percentage of seropositive donors to the virus is in the range of 1-15%. In another embodiment of the invention, the percentage of seropositive donors to the virus is in the range of 8-13%.

In another embodiment of the invention, the pathogenic virus is WNV.

In still another embodiment of the invention, the ELISA assay is carried out in an automated system.

A further aspect of the invention is to provide a composition for the treatment or prevention of a WNV associated disease, disorder or condition comprising blood or blood fractions obtainable by the screening method specified above.

In yet a further aspect, the invention provides a method for treating or preventing active encephalitis caused by West Nile Virus by administering to a subject in need a therapeutically effective amount of a composition according to the invention.

### Brief description of the drawings

**Fig. 1** shows anti-WNV titers in Arbitrary Units (AU/ml) in intravenous immunoglobulin (Omr-IgG-am^{™}) produced from Israeli plasma between 1998 and 2003.
**Fig. 2** shows anti-WNV titers in Arbitrary Units (AU/ml) in intravenous immunoglobulin (Omr-IgG-am^{™}) produced from Israeli plasma between 2004 and 2006.
**Fig. 3** shows the distribution of anti-WNV antibody titer in single positive plasma units in 2002.
**Fig. 4** shows the distribution of anti-WNV antibody titer in single positive plasma units in 2006.
**Fig. 5** shows survival among WNV-infected mice. Groups of 4-week old BALB/c mice were inoculated with 2 plaque forming units (PFU; diamond), 5 PFU (square), 20 PFU (triangle), or 100 PFU (cross) of West Nile Virus; strain NY 385-99 (NY99).
**Fig. 6** shows survival of 2-20 PFU inoculated mice plotted against the relevant inoculums.
**Fig. 7** shows the effect of IVIG administration on survival among mice infected with 20 PFU (4 X LD50) of NY99 WNV. Groups of 4-week old BALB/c mice were inoculated with WNV and 4 hours later the animals were given different quantities of WNIG (W), Israeli IVIG (IS), or US IVIG (US). The broken line represents survival of control animals receiving virus only.
**Fig. 8** shows the effect of IVIG administration on survival among mice infected with 100 PFU (20 X LD50) of NY99 WNV. Groups of 4-week old BALB/c mice were inoculated with WNV and 4 hours after challenge the animals were given different quantities of WNIG (W), Israeli IVIG (IS), or US IVIG (US). The broken line represents survival of control animals receiving virus only.
**Fig. 9** shows mean change of weight compared to day "0" among animals infected with 20 PFU (4 X LD50) of NY99 WNV and the effect of IVIG administration.
Groups of 4-week old BALB/c mice were inoculated with WNV and 4 hours later the animals were given different quantities of WNIG (W), Israeli IVIG (IS), or US IVIG (US). Mean weight was calculated for all surviving animals on each day.
**Fig. 10** shows mean change of weight compared to day "0" among animals infected with 100 PFU (20 X LD50) of NY99 WNV and the effect of IVIG administration.
Groups of 4-week old BALB/c mice were inoculated with WNV and 4 hours later the animals were given different quantities of WNIG (W), Israeli IVIG (IS), or US IVIG (US). Mean weight was calculated for all surviving animals on each day.
**Fig. 11** shows the effect of a low dose of IVIG administration on survival of mice after infection with 50 PFU (10 X LD50) of NY99 WNV. Groups of 5-week old BALB/c mice were inoculated with WNV and 4 hours later the animals were given a single injection of 0.05, 0.025 or 0.01 mg of WNIG or 0.25 mg IVIG-IS. Mortality was monitored for a period of 21 days.
**Fig. 12** shows the effect of late IVIG administration on survival among WNV-infected mice. Groups of 4-week old BALB/c mice were inoculated with 50 PFU of NY99 strain. Each group of animals was treated with 2 doses of 2 mg of WNIG on days 2 and 4 (diamonds); IVIG-IS on days 2 and 4 (Squares); WNIG on days 3 and 5 (triangles) or IVIG-IS on days 3 and 5 (asterisk). Mortality was monitored for a period of 11 days.
**Fig. 13** shows the effect of late IVIG administration on survival among WNV-infected mice monitored for a longer period of time. Groups of 5-week old BALB/c mice were infected with 50 PFU of NY99 strain and treated with 2 doses of 2 mg of WNIG or IVIG on days 2 and 4 or days 3 and 5. Mortality was monitored for a period of 21 days.

### Description of embodiments of the invention

In one aspect, the invention provides a method for the preparation of an immunoglobulin composition comprising a high level of neutralizing antibody specific for a pathogenic virus causing a new emerging disease, disorder or condition in a target geographic area, the method comprising screening blood or blood fractions of a plurality of donors of a population from a geographic area endemic to the virus, and isolating and pooling all blood or blood fractions of donors that are positive for antibody specific to the pathogenic virus.

The term "new emerging disease, disorder or condition" refers to an infectious disease, disorder or condition that has newly appeared in a particular geographic area. Such disease, disorder or condition may be known for some time but is rapidly increasing in incidence or geographic range.

The pool of selected blood or blood fractions can be processed to an intravenous immunoglobulin composition. In one embodiment of the invention, the viral strain used to screen positive donors in the endemic population is isolated from the target geographic area or nearby the target geographic area. In a further embodiment of the invention, the screening of blood or blood fractions comprising antibody specific to the pathogenic virus in the endemic population is carried out by a specific ELISA. The term "neutralizing antibody" refers to an antibody having a binding specificity to a particular antigen over other antigens. This antibody reacts with specific antigen(s) or determinant(s) of an infectious agent (usually a virus) and destroys or inhibits its infectivity and virulence. In one embodiment of the invention, the antibody binds an antigen associated with West Nile Virus and destroys or inhibits its infectivity and virulence.

The term "blood fraction" refers to a fraction of whole blood comprising immunoglobulins such as plasma.

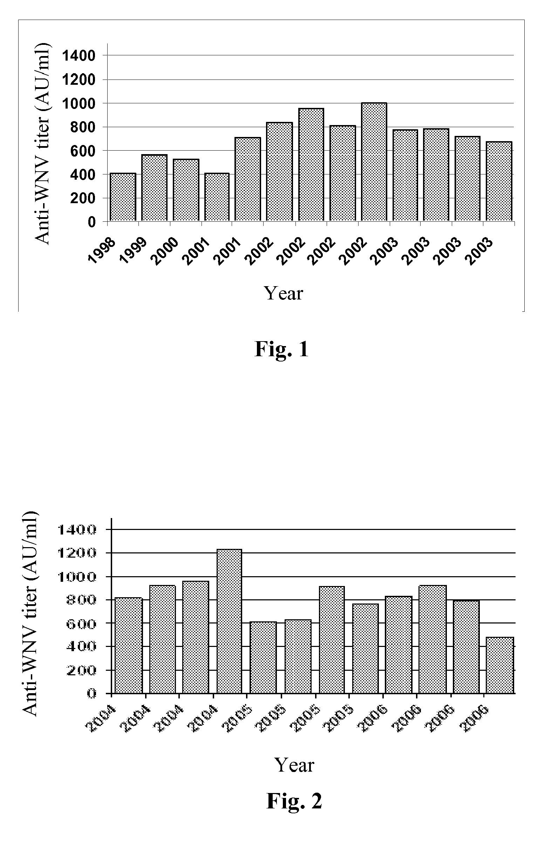

Immunoglobulin preparations prepared from plasma originated from donors of a population in a geographic area endemic to the West Nile Virus (WNV) contain anti-WNV antibodies. Since WNV has been endemic in Israel for many years, a portion of the population has a history of exposure to the virus. Thus, intravenous immunoglobulin (IVIG) prepared from regular (unscreened) plasma of pooled Israeli blood donors (e.g. Omr-IgG-am™) contains anti-WNV antibodies. It was found according to the invention that IVIG preparations produced from donors living in endemic region such as Israel but processed in different years or even within a single year contain variable ranges of WNV antibodies (Figs 1 and 2).

The method of the invention allows the preparation of a consistent and potent immunoglobulin composition (WNIG) for treatment or prevention of a West Nile virus related disease, disorder or condition. It was shown according to the present invention that WNIG comprises a high level of specific anti-WNV neutralizing antibody.

The method of the invention for producing a high titer immunoglobulin composition comprises screening blood or blood fractions of a plurality of donors from a population of a geographic area endemic to the WN virus that has a percentage of equal or less than 25% seropositives, such as Israel and neighboring territories, and isolating and pooling all blood fractions of seropositive donors for WNV antibody. In one embodiment of the invention, further processing of the pooled plasma to intravenous immunoglobulin composition is carried out. According to the invention, the population of the geographic area endemic to the virus has a percentage of positive donors that is not more than 25%, or in the range of 1-15% or 8-13% of the total of donors tested.

The term "area endemic to the virus" refers to a particular location, region, or people of a certain class in which the viral disease, disorder or condition is or was confined to or found in. The disease, disorder or condition can constantly exist in the particular location, region, or people of the certain class. Also, the disease, disorder or condition can have visible symptoms or hidden symptoms. The endemic disease, disorder or condition can be spread by vectors, through direct human contamination, or by any other way known in the field of epidemiology.

The pool of selected blood or blood fractions may be purified and/or concentrated using well known techniques for preparing IVIG solutions. Examples of such techniques are disclosed in U.S. Patent No. 6,468,733 (Nur and Bar, 2002) and International PCT Publication WO99/18130 (Nur, 1999) whose contents are incorporated by reference. The method disclosed in the above U.S. patent reduces the level of protein aggregates in the concentrated immunoglobulin solution and enables the final product to reach a level of 10% protein with a low level of aggregates for IV administration. For example, a method for the purification of immunoglobulins from a source solution such as Cohn Fraction II may comprise: (a) pre-treating a cation exchange resin with an acidic solution having a pH of 4.0-4.5; (b) contacting the source solution with the cation exchange resin; and (c) eluting the immunoglobulins bound to the cation exchange resin. Prior to contact with the cation exchange resin, the source solution may be treated with an organic solvent and detergent.

The term "positive donors or seropositive donors" relates to donors exhibiting detectable levels of antibody against the specific virus. In one embodiment the virus is WNV.

The presence of the specific antibody in the sample can be measured by a variety of methods such as a biochemical assay, a biological assay or by any other technique known in the field of immunology.

The method may be performed where the antigen is immobilized on a solid support such as an ELISA plate. In such an embodiment, the solid support to which the antigen is bound is contacted with the tested sample containing the antibody under conditions such that any specific antibody present in the sample binds to the bound antigen and forms a complex therewith, and any non specific antibody which is not bound to the complex is removed. The antigen used on the solid support can be inactivated, for example, by beta-propiolactone inactivation. The tested samples can be diluted in order to fit the linear range. The presence of antibody in the sample can then be qualitatively or quantitatively determined using a standard sample.

Several variations of ELISA testing may be employed, including indirect ELISA, sandwich ELISA, and competitive binding (Current Protocols in Immunology Copyright © 2007 by John Wiley and Sons, Inc. Last updated: 22 Feb 2008 Chapter 2.1). An automated ELISA system can be used in order to allow multiple screening, for example, by using an automated instrument.

After determining the titers of antibodies against WNV, all blood or blood fraction samples having detectable levels of antibody specific for WNV are pooled for the preparation of the high titer immunoglobulin composition.

A detectable level can be a minimal value or cut-off which is considered positive.

The minimal value is defined as 40% of the middle range of the linear curve multipled by the dilution required to bring the sample to the linear response range. For example, when the dilution required in order to remain within the linear range is 1:50 and the linear range is 1-10 AU/ml, the minimal value is 100 AU/ml. All plasma units having a titer lower than 100 AU/ml are considered negative.

In an embodiment of the invention, a standard sample with approx. 100 AU/ml is designated as "low-positive". The low-positive standard sample is tested at 1:50 dilution in order to remain within the linear range and each blood or blood fraction sample is diluted 1:100. The diluted samples and standard are measured by spectrophotometer at 405 nm. Each sample having an Optical Density (OD) higher than the "low-positive" sample is considered positive.

In another embodiment of the invention, the cut-off is 100 AU/ml, as measured by ELISA as exemplified below.

The term "Arbitrary Units" refers to an arbitrary antibody titer measure, calculated using the ELISA immunoassay such as the one described below.

ELISA can be carried out as follows: 100 µl of inactivated WNV (NY99) (beta-propiolactone inactivation; final concentration of 0.001 %) was diluted (1:500) in carbonate buffer (pH=9.6), bound to the surface of a microtiter plate and incubated overnight at 4°C. Afterwards, the coating buffer was decanted, and the plates were washed three times with washing buffer (PBS containing 0.5% Tween 20 and 0.02% sodium azide). The plates were then blocked for 1 hour at 37°C with 200 µl of blocking buffer (0.5% I-block and 10% goat serum). After three additional washes, 100 µl of the samples and the controls were added. Samples containing the specific immunoglobulin G (WNV antibodies) such as serum or plasma samples, calibration standards and controls were diluted in blocking buffer in order to fit the standard curve range, pipetted into the wells and incubated for 1 hour at 37°C in a humidified atmosphere. A positive control (650 AU/ml) and a negative control (<50 AU/ml) were run in each plate. All measurements were tested in duplicates. WNV antibodies present in the sample bind to the immobilized WNV inactivated antigen. Unbound material was removed by three washes. In a second step, 100 µl Goat anti Human IgG conjugated to Alkaline Phosphatase diluted 1:1000 in blocking buffer was added into the wells and the ELISA plate was incubated for 1 hour at 37°C in humidified atmosphere. After incubation, unbound conjugate was removed by 3 washes. The enzymatic activity of Alkaline Phosphatase was revealed after 1 hour incubation at room temperature by its reaction with 100 µl p-nitrophenyl phosphate substrate. The measurement was done photometrically at 405 nm. The titer of each serum and control sample was calculated by using the absorbance at 405 nm plotted against the log transformation of calibrator concentration.

To allow quantitative measurements, specific positive plasma was designated as a calibration standard and assigned a value of 2000 AU/ml of WNV antibodies. This plasma was diluted, and the range of 1-10 AU/ml was found to have linear characteristics when the log-transformation of concentration was related to the observed optical density at 405 nm. This range was used as a working range to establish a linear standard curve for quantification of unknown samples.

In another further embodiment of the invention, a bioassay such as plaque reduction neutralization tests (PRNTs) can be used in order to determine the positive units.

The term PRNT refers to a reduction in plaque counts, e.g. the PRNT titer PRNT50 is based on a 50% or greater reduction in plaque counts. The assay can be performed in an *in-vitro* setting in various cell types, for example Vero cells isolated from kidney epithelial cells extracted from African green monkey.

The PRNT titers can be calculated by counting plaques and reporting the titer as the reciprocal of the last serum dilution to show >50% reduction of the input plaque count as compared to the control input plaques.

The PRNT assay can be very cumbersome and might not be adapted to high throughput samples. This assay involves a fully equipped virological lab with the capabilities of continuous culturing of viruses and cell culture in a highly classified control environment.

Animal models for WNV infection can be used in order to assess the *in-vivo* efficacy of the immunoglobulin preparation in preventing and treating West Nile Fever (WNF), such as the mouse lethal model. For example, in the mouse lethal model, 4-5 weeks old mice can be inoculated intraperitoneally (IP) with different amounts of stock virus. The WNV challenge dose can be expressed as a multiplication of the dose required to kill half the members of the tested population (Lethal dose 50; LD50) which can be calculated by an Excel software or by any other well known technique. The treatment or prevention procedure can be carried out in the animal model as follows. In a post-exposure prophylactic animal model of WNV infection, the immunoglobulin preparation can be given IP four hours after challenge with WNV. In a treatment model, the immunoglobulin preparation can be given IP on day 2 post WNV challenge when the viremic phase reaches a peak, or on days 3-4 post challenging when an infection of the central nerve system (CNS) occurs.

As mentioned, the method for producing the high titer immunoglobulin composition of the invention can comprise screening blood or blood fractions of a plurality of donors from a population of a geographic area endemic to the virus and isolating and pooling all positive positive plasma units.

According to the invention, highly efficacious immunoglobulin can be produced from donors living in WNV endemic regions by selection of plasma from the portion of donors with detectable WNV antibodies. According to the invention, the percentage of positive donors in the population of the geographic area endemic to the virus is not more than 25%, or in the range of 1-15% or 8-13% of the total of donor tested. In another further embodiment of the invention, the percentage of positive donors in the population of the geographic area endemic to the virus is about 10%.

Screening of positive donors for WNV can be carried out employing a virus isolated from a target geographic area or nearby. A target geographic area is, for example, an area in which a WNV epidemic is expected or there is spread of WNV. A nearby area is an area neighbouring the target area and can be located e.g. in the same country as the target area or same continent. In one embodiment of the invention, the composition is intended for use in an area of the United States in which the virus causes a new emerging disease, disorder or condition (the target geographical area), and the screening of positive is carried out with Israeli donors using a virus strain isolated from said area of the United States or a neighbouring area of the target area in the USA. Thus, in this case the plasma donors are from a geographic area endemic to the virus whereas the virus used for screening of positive donors is originated from a target area or nearby, which is far away (e.g. a different continent) from the geographical area of the donors.

The geographic endemic area and the target area can be located within the same territory, wherein the virus is endemic in some regions of that territory, for example, the endemic area and the target area can both be from different and neighboring areas of the USA territory.

In another aspect, the invention provides an immunoglobulin composition (e.g. WNIG) obtainable by the above method(s) of the invention. For example, the invention provides an immunoglobulin composition comprising immunoglobulin from a pool of plasma donors of a geographical area endemic to the West Nile virus, like Israel and neighboring territories, wherein all the donors in the pool are positive for West Nile virus, such as WNV isolated from the target area, for example the an area located at the United States. The high titer immunoglobulin composition of the invention has the advantage that it is more consistent and potent than regular (unscreened and pooled) immunoglobulin composition from an endemic area to the virus, since the high titer immunoglobulin composition is obtained from pooling individually screened and selected blood or plasma positive for WNV antibody against the specific virus which may be from the target geographic area or near the target area. The high titer immunoglobulin composition of the invention has at least 5 times, about 10, or more than 10 times higher titer, such as 11 times higher titer, of anti WNV neutralizing antibody compared to regular immunoglobulin composition from a pool of un-selected donors from, or living in a geographic area endemic to the virus and therefore is more potent. In one embodiment of the invention, the levels of antibodies in the high titer immunoglobulin composition specific for WNV are at a titer of greater than or equal to 7600 AU/ml when measured using an ELISA assay as exemplified below and/or have a neutralizing activity of greater than or equal to 9900 WNU/ml when measured using PRNT50 assay as exemplified below.

The use of a high titer immunoglobulin composition, having high titer and being more potent than regular un-selected endemic immunoglobulin, is also advantageous because, if needed, more WNV specific neutralizing active antibodies can be administered in a reasonable volume via intravenous, intramuscular, or subcutaneous route. Also, titers of the immunoglobulin composition of the invention which are similar to unscreened IVIG can be administered in smaller volumes and therefore can be easily used under field conditions by any trained emergency personnel. The high titer immunoglobulin composition of the invention can be formulated in small and easy to use pre-filled syringes or vials.

In one aspect, the invention relates to a method for treating or preventing a WNV associated disease, disorder or condition comprising administering to a subject in need an immunoglobulin composition of the invention. A WNV associated disease, disorder or conditions includes, but is not limited to, West Nile Fever , flu-like symptoms such as fever, headache and possibly a rash, West Nile Neurological Syndrome, neuroinvasive disease, including meningitis, encephalitis, or acute flaccid paralysis. People who contract WNV usually experience only mild symptoms - fever, headache, body aches, skin rash, and swollen lymph glands. However, if the virus penetrates the brain, it can cause life-threatening encephalitis or meningitis.

The immunoglobulin compositions of the invention can be administered by routes that lead to systemic absorption. Non limiting examples of administration routes include, but are not limited to, intravenous, subcutaneous, intraperitoneal, and intramuscular. The administration can be carried out in an initially higher dose followed by the same or lower doses at intervals. The higher doses can be intended to rapidly increase the patient's immunoglobulin concentration to an efficacious target concentration.

The term "intravenous" refers to administration of the composition into the vein of an animal or human patient. The administration can be intermittent or by continuous dripping. The term "intermittent" is synonymous with the term "intravenous bolus" or "intravenous push".

The term "subcutaneous" refers to introduction of the composition by injection under the skin of an animal or human patient. The injection can be carried out by creating a pocket such as by pinching or drawing the skin up and away from underlying tissue. Optionally, the infusion may be carried out by subcutaneous implantation of a drug delivery pump implanted under the skin of the patient. The pump can deliver a predetermined amount of the immunoglobulin at a predetermined rate for a predetermined period of time.

By "intramuscular" is meant an introduction of the immunoglobulin directly into a muscle. The injections can be given into any muscle including, but not limited to, the deltoid, vastus lateralis, ventrogluteal and dorsogluteal muscles. The administration can be carried out at multiple locations.

"Intraperitoneal injection" refers to the injection of the immunoglobulin into the peritoneum. The method can be used in animals.

In principle, smaller volumes could be achieved also by using Hyperimmune immunoglobulins selected from vaccinated subjects (US Patent Publication No. 20070037170 (Nur et al. 2007)). However, plasma selected from positive donors from endemic areas can be superior to plasma from vaccinated donors since plasma from a healthy subject recovering from a large variety of infectious viral permutations produces a humoral response that "cured" them of infectious agent. This approach is also of advantage when vaccine is not available as in the case of West Nile Virus. On the other hand, many endemic viruses, such as hepatitis virus and CMV, can be found at high percentages in the population and therefore further selection of blood or plasma of positive donors and pooling plasma from all positive donors may not be of further advantage. In fact, we found according to the invention that in Israel only 8%-13% of the individual plasma units were positive for anti-WNV antibody. These results indicated that it is possible to develop a highly potent preparation of immunoglobulin composition for treating or preventing WNV associated disease based on selecting and pooling all blood and/or blood fractions from a small percentage of donors positive for the virus in an endemic area. In one embodiment of the invention, the selection for positive Israeli donors is carried out employing a West Nile virus strain isolated from a target area in the United States. The high titer immunoglobulin composition of the invention has more than 2 times, at least 5 times, about 10 or more than 10 times higher titer, such as 11 times higher titer, in anti WNV neutralizing antibody compared to regular non-screened IVIG from a pool of un-selected donors from, or living in, a geographic area endemic to the virus and therefore is more potent than non-screened IVIG. Also, a preparation selected in this way provides more consistent and better performance in a target area.

The method of the invention enables selection of WNV neutralizing blood or blood fractions resulting in highly potent immunoglobulin composition. Thus, more potent immunoglobulin composition can be provided in lower volume unit dosage form, such as a vial or a pre-filled syringe.

The term "low volume unit dosage form" refers to a dosage form which can be administered to a patient in a single dispensation, usually by injection. In this form, the patient can receive a complete individual treatment in one, brief administration. Thus, in another aspect, the invention provides a vial or pre-filled syringe containing a low-volume unit dosage of the composition according to the invention. Non limiting examples of volumes of the high titer immunoglobulin composition of the invention which can be used in therapy are 0.5 ml, 2 ml, 10 ml, 30 ml, 50 ml, 100 ml, 500 ml, 1 liter, 2 liter and 3 liter.

Indeed, it was shown according to the invention that the high titer immunoglobulin composition of the invention is superior to regular Israeli IVIG in prophylaxis as well as in treatment of established disease in an animal model inoculated with different concentrations of NY99 WNV.

The high titer immunoglobulin composition according to the invention contains at least about 11 and 10 times more anti-WNV antibodies than regular Israeli IVIG as measured by both ELISA and Plaque neutralization activity, respectivly. These *in vitro* results were consistent with the high efficacy of WNIG in preventing and treating WNV infection in the mice model.

Survival of animals receiving 100 PFU (20 X LD50) and 20 PFU (4 X LD50) was used as a measurement of the efficacy of IVIG in prophylaxis of WNV infection. In both cases the US-source IVIG gave very moderate protection when given at the highest dose of 2.0 mg/mouse (equivalent to 120 mg/kg). In the case of WNIG, complete protection was achieved with as low as 0.1 mg/mouse (equivalent to 6 mg/kg).

Regular IVIG from Israeli donors gave relatively good protection ranging between 75-87% with 0.5 mg/mouse (equivalent to 30 mg/kg) but had only 12% protection at the lowest dose of 0.1 mg/mouse. Thus, treatment with the high titer immunoglobulin composition of the invention is more effective since it is at least 5 times more potent than regular IVIG and at least 20 times more potent than US-source IVIG; consequently administration of lower doses is possible.

Thus, the invention provides a pharmaceutical composition for the treatment or prevention of a WNV associated disease, disorder or condition comprising a pharmaceutically acceptable carrier and an immunoglobulin composition comprising immunoglobulin from a plurality of donors of a population of a geographical area endemic to the virus, wherein all the donors contain a detectable titer of anti WNV antibody to WNV, particularly to WNV strain from the target area, thereby having a consistent high titer and high levels of anti WNV neutralizing antibody which are more than 2 times, at least 5 times or about 10 or more than 10 times higher than those of regular IVIG from a pool of un-selected donors from the geographic area endemic to the virus.

The term "pharmaceutically acceptable carrier" refers to a carrier which is suitable for an intravenous, subcutaneous and/or intramuscular injection. Non-limiting examples of carriers are: saline, water, emulsions and mixtures of organic solvents.

The immunoglobulin composition can also comprise excipients. As used herein the terms "excipient" refers to an inert substance which is added to the pharmaceutical composition. Examples of excipients include, but are not limited to, various sugars, such as maltose or sucrose, or serum proteins, such as albumin.

The immunoglobulin composition is purified from infective particles. The purification procedure can be carried out by filtration, solvent/detergent treatment, heat treatment, such as, but not limited to, pasteurization, gamma or UVC (< 280 nm) irradiation, or by any other method known in the art.

The term "infective particle" refers to a microscopic particle, such as, but not limited to, a microorganism or a prion, which can infect or propagate in cells of a biological organism. The infective particles can be viral particles.

The inactivation procedure of infective particles can be carried out by adding an inactivating molecule to the composition prior to and/or during the purification procedure. The added molecules and their products can be removed by gravitation, column chromatography or by any other method known in the art. The removal of infective particles can be carried out by filtration or by selective absorption methods such as affinity, ion exchange or hydrophobic chromatography.

A multi-step viral inactivation procedure can be carried out. For example, the composition can be subjected to solvent/detergent treatment, heat treatment, selective chromatography and filtration.

Survival of animals receiving 50 PFU (10 X LD50) was used to assess the efficacy of 2 mg/mouse (equivalent to 120 mg/kg) IVIG on day 2 and 4 or 3 and 5 post WNV infection. WNIG showed complete protection when treatment was given on day 2 post infection. Regular IVIG achieved relatively good protection with about 75% survival. Treatment of mice 3 and 5 days post infection, in the encephalitis phase, with WNIG postponed the average death day and achieved a higher survival rate compared to non-screened Israeli plasma. Thus, treatment with the high titer immunoglobulin composition of the invention during active encephalitis is effective and enhances the survival of the animals as compared to IVIG-IS.

When higher quantities of WNIG, 8 mg/mouse (equivalent to 480 mg/kg), were given on day 3 or on days 3, 5 and 10 post challenge with 50 PFU (single or triple injections, respectively) complete protection was achieved. Also, treatment with 8 mg/mouse WNIG on day 4 or on days 4, 6 and 10 post challenge with 50 PFU (single or triple injections, respectively) achieved relatively good protection with about 69% survival compared to 25% of the un-treated group.

Also, the invention provides a method of treatment or prevention of a WNV associated disease, disorder or condition comprising administering to a subject in need a therapeutically effective amount of an immunoglobulin composition comprising immunoglobulin from a plurality of donors of a geographical area endemic to the virus, wherein all the donors contain a detectable titer of anti WNV antibody. In one embodiment the donors are positive for antibodies to a viral strain from or nearby the target geographical area.

The term "therapeutically effective amount" refers to the dose required to prevent, inhibit the occurrence, or treat (relieve a symptom or all of the symptoms) the disease, disorder or condition. The effective amount can be measured based on any change in the course of the disease in response to the administration of the composition. The effective dose can depend on the route of administration, the phase of the disease (e.g., early or advanced) and other factors which can be recognized by the skilled artisan.

It is one object of the invention to provide a method of treatment or prevention of a WNV associated disease, disorder or condition comprising administering lower protein amounts than the standard protein amounts of IVIG. The invention contemplates the administration, for example, of equal or less than 0.4, or 0.1-0.2, 0.2-0.3, 0.04-0.2, 0.01-0.04, or 0.02-0.2 g/kg of an immunoglobulin composition according to the invention to a subject in need.

In some difficult cases when using a common regular IVIG from an endemic region, very high levels of active antibody, and thus very high volumes and very high protein quantities (more than 0.4 g/Kg), are needed.

A WNV associated disease, disorder or condition may require administration of unreachable excessive amount or volume of IVIG from a regular pool of donors of the geographic area endemic to the virus. The maximum allowed amount of protein that can be administered is of about 2 g/kg.

The composition according to the invention solves this technical problem since it was found to be significantly more potent than the regular IVIG allowing administration of more reasonable volumes and protein quantities.

The transport of antibodies across the blood brain barrier (BBB) is inefficient and the possibility to treat or prevent active encephalitis is of major concern. During active infection the BBB becomes partially permeable, thus allowing the penetration of significant quantities of antibodies to the infected site. Using an immunoglobulin composition which comprises high level of neutralizing antibody directly increases the specific antibody levels in the brain even before active disease in the BBB and thus can prevent active encephalitis.

Thus, also subject matter of the invention is a method for treating or preventing a WNV associated disease, disorder or condition which requires administration of what would have been an unreachable excessive amount or volume of immunoglobulin from a regular pool of donors of the geographic area endemic to the virus, the method comprising administering to a subject in need a therapeutically effective amount of an immunoglobulin composition according to the invention.

It was shown according to the invention that an immunoglobulin preparation or composition comprising immunoglobulin from a plurality of donors which all of them contain a detectable titer of antibodies to West Nile Virus NY99 WNV, having more than 2 times, at least 5 times, about 10 or more than 10 times higher titer, and about 11 times higher titer and level of anti WNV neutralizing antibody compared to regular non-screened IVIG from a pool of un-selected donors from, or living in a geographic area endemic to the virus, can be used as a consistent and potent preparation for treatment or prevention of WNV infection, WNF and/or encephalitis induced by WNV. In one embodiment an Israeli high titer immunoglobulin composition was produced by selection of positive titers in Israeli plasma donors with a non local West Nile virus to increase the probability that the preparation will be equally effective for a non Israeli affected population.

It was surprisingly found according to the present invention that 0.1 mg/mouse WNIG had similar protective efficacy as 0.5 and 2 mg/mouse IVIG from Israeli pooled plasma and that 0.025 and 0.05 mg/mouse WNIG and 0.25 mg/mouse IVIG from Israeli pooled plasma lead to similar survival level.

Thus, the invention provides high titer immunoglobulin compositions or preparations that are more than 2 times, at least 5 times, or about 6, 7, 8, 9, 10, 11, 12, 13, 14, 15, 16, 17, 18, 19, or 20 times more potent against WNV in a target area compared to regular IVIG from an endemic area, such as Israel and neighboring territories.

The anti-WNV antibody levels of the high titer immunoglobulin composition of the invention was 10-11 folds higher than regular IVIG from Israeli plasma as measured by the neutralization activity and by ELISA. For example, a preparation of regular IVIG prepared in 2003 had a titer of 668 Arbitrary Units (AU)/ml and a neutralizing activity of 1032 PRNT50 West Nile Units (WNU)/ml while the high titer immunoglobulin composition prepared according to the invention had a titer of 7608 AU/ml and a neutralizing activity of 9900 PRNT50 WNU/ml.

Yet in another aspect, the invention provides an efficient method for screening and selection of blood or blood fraction comprising high level of neutralizing antibody specific for a pathogenic virus causing a disease, disorder or condition from numerous blood or blood fraction samples comprising the steps of: screening blood or blood fraction samples obtained from donors of a geographic area endemic to the virus using an ELISA assay; and selecting positive blood or blood fractions of donors that are positive for said pathogenic virus.

The positive blood or blood fractions refer to a detectable level or cut-off as specified above. In one embodiment of the invention, the cut-off titer is 10 AU/ml, 20 AU/ml, 30 AU/ml, 40 AU/ml, 50 AU/ml, 60 AU/ml, 70 AU/ml, 80 AU/ml, 90 AU/ml or 100 AU/ml, as measured by ELISA as exemplified below.

In another embodiment of the invention, the cut-off is 100 AU/ml, as measured by ELISA as exemplified below.

According to the invention, the geographic area endemic to the virus exhibits not more than 25%, or in the range of 1-15% or 8-13% of specific antibody, such as 10% of the total of blood or blood fractions tested.

Yet, in another further embodiment of the invention, the pathogenic virus is WNV. Several variations of ELISA testing can be used in order to determine level of neutralizing antibody in the plasma units, including, but not limited to, indirect ELISA, sandwich ELISA, and competitive binding. Also, an automated ELISA system can be used, for example by using an automated instrument, in order to allow multiple screening.

Subject matter of the present invention is also a pharmaceutical composition for the treatment or prevention of a WNV associated disease, disorder or condition using a blood or blood fraction screened by the above specified method.

The following are advantages of the intravenous immunoglobulin composition (WNIG) of the invention as anti-viral therapy specific to WNV: the intravenous immunoglobulin composition is more consistent and up to 20 times more effective than regular IVIG from endemic area and therefore is more suitable for treating or preventing a disease, disorder or condition caused by WNV (such as encephalitis and meningitis) in an infected area and can serve as an immediate countermeasure to the expected epidemic and spread of WNV; more immunoglobulin composition (e.g., 5 to 10 to 20 times more potent immunoglobulin) can be administered in a suitable volume, e.g., the intravenous immunoglobulin composition (WNIG) may be administered in up to 3 liter for treating more severe cases, such as immunocompromised WNV infected patients; low volume-aliquots of the intravenous immunoglobulin composition (WNIG) can be prepared for easy administration, for example, by non trained personnel.

There is a need for the rapid development of a therapy to treat emerging epidemic diseases caused by a new virus. Thus, the results obtained according to the invention have far-reaching consequences in terms of other currently untreatable new emerging diseases caused by viruses. Isolation and recognition of the new virus causing the disease, and development of diagnostic tests such as ELISA, bioassay or based on specific PCR for the specific pathogen followed by the method of preparation including screened blood or plasma as generally thought by the invention can be used for rapid development of a therapy for the new disease, disorder or condition caused by a viral pathogen.

According to the invention, un-screened IVIG from a WNV endemic area is not efficient for treatment against severe WNV infection since the level of specific antibodies in the IVIG is too low. In contrast, the high titer immunoglobulin composition of the invention, which is produced based on selection of plasma units from a small fraction of blood donors, contains high level of neutralizing antibody specific for WNV, resulting in a highly efficient composition for prophylaxis or treatment of WNV encephalitis.

The concept of the method of the invention can be used to find therapy for diseases caused by additional pathogenic viruses, such as, Flaviviruses, for example , Yellow Fever and Dengue; arenaviruses, for example Junin, Guanarito, Sabia, Machupo, WhitewaterArroyo, Lassa and LCMV; Filoviruses, for example Ebola and Margburg; and other viruses such as HCV, RSV, and Infuenza. IgG preparations according to the invention could then be developed from selected plasma samples and formulated for convenient IV or IM administration. This method provides the basis for the treatment of a number of dangerous and previously untreatable viruses.

Vaccination (with an active vaccine) and passive immunization are two complementary approaches that can be used together to treat an emerging disease. Vaccination may be used as a prophylactic measure to protect at-risk populations, while the high titer immunoglobulin composition of the invention may be used to treat patients who are already infected or as a prophylactic alternative to populations when immediate protection is needed or for subjects excluded from a vaccination program, e.g., infants, pregnant women, patients receiving immunosuppressive treatment, or other immunocompromised patients. Indeed, administration of WNIG of the invention to immunosuppressed mice challenged with WNV resulted in complete protection (see Table 6).

The disclosure of ranges in the description of the invention is easily understood by the skilled person. It means the disclosure of continuous values and figures between the limits of the ranges, including the limiting figures and values.

The disclosure of applications, patents and publications, cited above or below, is hereby incorporated by reference.

The following examples are illustrative but not limiting.

### Examples

### Materials and Methods

**WNV Antigen preparation for ELISA Assay:** Vero cell line (ATCC #CRL-1587), originally derived from kidney of normal African Green Monkeys, was seeded at a concentration of approximately 5x10⁶ cells per ml in DMEM with 4.5 g/l glucose, supplemented with 10% FCS, 2mM L-Glutamine, 1mM Sodium pyruvate, 0.15% Sodium bicarbonate, 1% Penicillin-Streptomycin-Amphotericin B solution, 50 µg/ml Gentamycin sulfate and Nonessential amino acids, and incubated over night at 37°C, 5% CO₂. On the next day, the medium was replaced with WNV (1x10⁵ PFU/ml diluted in MEM supplemented with 2% FCS and as indicated above) and returned to the incubator until a cytopathic effect (CPE) was observed (when about 50% of the cells were detached). Then, the cells and medium were collected and subjected to centrifugation at 1000 g for 10 min at 4°C. The precipitated cells were washed three times with wash solution (50 mM Tris, 50 mM EDTA, 150 mM NaCl, pH 7.2) and then re-suspended in TE buffer (50mM Tris, 0.5mM EDTA; pH 8.7) (1 ml per 185 cm² flask). The solution was then subjected to three cycles of freezing and thawing and to centrifugation at 20,000 g for 15 min at 4°C. The supernatant, which contains the virus, was collected. The medium left from the first centrifugation was subjected to ultracentrifugation at 40,000 rpm (rotor T-647.5) for 2 h at 4°C. The supernatant was discarded and the pelleted virus was re-suspended in PBS (1 ml per 50 ml medium). The virus produced from the cells and from the medium was combined and inactivated with beta-propiolactone (0.001% final concentration; Serva, cat No. 33672.01). Inactivation was confirmed by plaque assay and/or in 3 days old suckling mice.

**ELISA:** 100 µl of inactivated WNV (NY99) (prepared as described in the previous paragraph) was diluted (1:500) in carbonate buffer (pH=9.6), bound to the surface of microtiter plate and incubated overnight at 4°C. Afterwards, the coating buffer was decanted, and the plates were washed three times with washing buffer (PBS containing 0.5% Tween 20 and 0.02% sodium azide). The plates were then blocked for 1 hour at 37°C with 200 µl of blocking buffer (0.5% I-block (Tropix, Bedford, MA) and 10% goat serum (Biological Industries, Israel, Cat. No. 04-009)). After three additional washes, 100 µl of the samples and the controls were added. Samples containing the specific immunoglobulin G (WNV antibodies), such as serum or plasma samples, calibration standards and controls, were diluted in blocking buffer in order to fit the standard curve range, pipetted into the wells and incubated for 1 hour at 37°C in a humidified atmosphere. A positive control (650 AU/ml) and a negative control (<50 AU/ml) were run in each plate. All measurements were tested in duplicates. WNV antibodies present in the sample binds to the immobilized WNV inactivated antigen. Unbound material was removed by three washes. In a second step, 100 µl Goat anti Human IgG Alkaline Phosphatase conjugate (Sigma Cat. No. A-3150) diluted 1:1000 in blocking buffer was added into the wells and the ELISA plate was incubated for 1 hour at 37°C in humidified atmosphere. After incubation, unbound conjugate was removed by 3 washes. The enzymatic activity of Alkaline Phosphatase was revealed after 1 hour incubation at room temperature by its reaction with 100 µl p-nitrophenyl phosphate substrate (Sigma Cat. No. N-2770). The measurement was done photometrically at 405 nm. The titer of each serum and control sample was calculated by using the absorbance at 405 nm plotted against the log transformation of calibrator concentration.

The assay was developed for quantitative and qualitative measurement of WNV antibodies in serum/plasma solutions. To allow quantitative measurements, specific positive plasma was designated as a calibration standard and assigned a value of 2000 Arbitrary Units per ml (AU/ml) of WNV antibodies. The range of 1-10 AU/ml was found to have linear characteristics when the log-transformation of concentration was related to the observed optical density at 405 nm. This range was used as a working range to establish a linear standard curve for quantification of unknown samples. The quantitative ELISA for WNV antibodies was validated as part of an effort to ensure the desired quality of the diagnostic tests.

The screening assay was designed to produce, for each plasma unit, two types of data: (a) a qualitative parameter that was used for screening (cut-off, yes/no) and (b) a quantitative parameter (similar to the one used in the unmodified assay) that was used for quantitative assessment and statistics.

**Cut-off determination:** In order to simplify the decision algorithm (that should be applied by the robot software), a standard sample with approx. 100 AU/ml was designated as "Low-positive". The low-positive standard sample was tested in duplicates at 1:50 dilution while the plasma samples were tested at 1:100 dilutions (singles to allow large number of tests). The OD of the standard was considered as a cut-off value and each plasma sample having an OD higher than the cut-off was considered positive. Samples that were defined as positives or negatives by the cut-off analysis were re-tested at the laboratory by the in-house validated ELISA to verify the result and get a precise quantified value for statistic assessment. 163 samples that were above the cut-off OD were re-tested by the validated assay and 7 samples (4.3%, CI95 1.9%-8.3%) had lower than 100 AU/ml (false positive). Eighty samples that were found negative by the cut-off definition were re-tested and none had more than 100 AU/ml.

**Cell Cultures:** Vero cell line (ATCC #CRL-1587) was propagated as described above. The cells were maintained in a humidified atmosphere at 37°C in 5% CO₂ and were used for growing virus stocks and virus titration.

**Plaque Reduction Neutralization Titer 50% (PRNT50):** The CPE of WNV on Vero cells can be neutralized by the presence of specific anti-WNV antibodies. This sensitive test quantifies the sample (containing WNV antibodies) dilution that results in 50% reduction of plaques relative to the virus control. The titer of neutralizing antibodies was determined essentially using a plaque reduction test as described by Ben Nathan, 2003. Briefly, Vero cells were seeded in a 6-well plate at a concentration of 5x10⁵ cells per well as described above and incubated overnight at 37°C, 5% CO₂. Two fold serial dilutions of the test sample were prepared in MEM supplemented with 2% FCS as above. Each dilution was mixed with an equal volume of about 450 PFU/ml of WNV NY-99 (diluted in MEM, 2% FCS). The sample mixtures were rolled overnight at 4°C. Four hundred microliters from each sample mixture was added to the wells and allowed to adsorb for 60 min at 37°C, 5% CO₂. Tragacanth-medium (Sigma Cat. No. G-1128) was mixed with an equal volume of X2 MEM medium supplemented with 4% FCS, 4 mM L-Glutamine, 2 mM Sodium pyruvate, 2% Penicillin-Streptomycin-Amphotericin B solution, 0.1 mg/ml Gentamycin sulfate and Nonessential amino acids, and Sodium bi-carbonate was added to the mixture to a final concentration of 0.15%. Three ml from the Tragacanth mixture was added to each well and the plate was incubated for 72 hours at 37°C, 5% CO₂. The cells were then fixed with 95% ethanol for 2 min (Riedel de Haen cat. No. 32221), stained with Fuchsin for 2 minutes (Sigma cat. No. P1528) and the plaques were counted.

The PRNT50 (expressed in WNU/ml) is the sample dilution at which there is 50% neutralization of WNV plaques as determined by the non-linear relationship (according to the logit model) between the In sample dilution and the percentage of plaque neutralization relative to the control sample (taken as 100%). The in-vitro assay for determination of the activity of neutralizing antibodies in cells exposed to WNV was validated as part of an effort to ensure the desired quality of the diagnostic tests.

**Experimental Methods-animal studies:** The experimental protocol was written and supervised by Harlan Laboratories, Israel, Ltd. (ISO 9001:2000 Certificate No.: US2002/3081). The study was reviewed and approved by the Committee for Ethical Conduct in the Care and Use of Laboratory Animals.

**Animals**: Female BALB/cOlaHsd mice (Harlan Laboratories, Israel) at the age of 3-7 weeks were used as WNV infected models. Weight variation of animals at the time of treatment initiation did not exceed ± 20% of the mean weight. The health status of the animals used in the study was examined on arrival. Only animals in good health were acclimatized to laboratory conditions (as specified below) and were used in the study.

**Housing:** During acclimation (at least 5 days) and throughout the entire study duration, the animals were housed in a P3 rodent facility at the Veterinary Institute, Virology Department, Beit-Dagan, Israel (Ministry of Agriculture, Israel) with free access to water and food (a commercial rodent diet). The animals were kept in groups (maximum 10 animals per cage) in polypropylene cages (37.5 x 21 x 18 cm), fitted with solid bottoms and filled with wood shavings as bedding material. The animal room has an automated controlled environmental condition system set to 20-24°C with a relative humidity (RH) of 30-70%, a 12:12 hour light: dark cycle and 15-30 air changes/hr.

**Allocation to treatment groups:** On Study Day 0, animals were assigned to treatment groups in a way that the mean body weight values were as similar as possible among all groups. Each treatment group was kept in a single cage and each animal within a cage was given a unique number.

**Study termination:** At the end of the study, surviving animals were euthanized by CO₂ asphyxiation.

**Virus challenge strain and virus titrations:** The virus used to infect mice in these studies was a third passage in Vero cells of West Nile Virus, strain NY 385-99 (WNV-NY99) (Steele EK et al., 2000; Xiao SY et al. 2001). The original virus was provided by M.S. Diamond, Washington University, School of Medicine, St. Louis, USA. Virus titers were expressed as plaque-forming units (PFU)/ml. Stock concentration: 5-6×10⁸ PFU/ml in TE 8.7/PBS (1:1.8), stored in aliquots at -70°C. Virus plaque assays were performed on Vero cells as described above. On day 0, the viral stock was thawed on ice and the challenge solutions (1000, 100, 50, 20, 10 and 5 PFU/ml) were prepared by dilution in DMEM, 2% FCS. The vials containing the final solutions were kept on ice until administration. The stock solution was restored at - 70°C for titer verification.

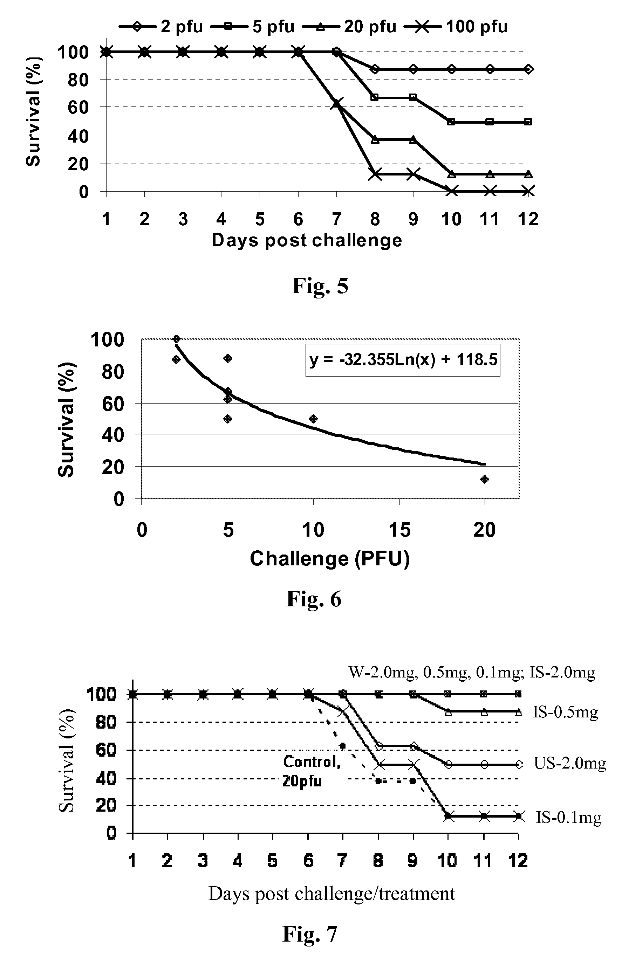

In all experiments (unless indicated otherwise) 4-5 week old mice were inoculated by a single intraperitoneal injection (IP; 0.2 ml/mouse) with different amount of stock virus by using a 1 ml insulin syringe and 25-27G needle. Lethal dose (LD50; the dose required to kill half the members of the tested population) was calculated by plotting the survival data in 2-20 PFU inoculations against the relevant inoculation and inserting a logarithmic trend line using Excel software (see Example 6; Fig 6). Four hours after challenge (unless indicated otherwise), the animals received an IP dose of 0.1 ml/mouse of the IVIG test material (specified below) using a 1 ml insulin syringe and 25-27G needle. Individual weights were usually taken every other day.

**Test materials:** All IgG preparations contained 5% IVIG solution in 10% maltose consisting more than 99% IgG and small quantities of IgA and IgM. Three types of test articles were used: (a) High titer WNV IVIG (WNIG), Batch #K44G511. This product was prepared by screening plasma units of Israeli donors by ELISA for IgG. About 10% of the donor population with antibodies for WNV had more than 100 AU/ml anti-WNV antibodies thus pooled and processed into WNIG; (b) Commercial IVIG, batch #H23131, prepared in 2003 from plasma of pooled Israeli blood donors; (c) Commercial IVIG, Batch #K30G370, prepared in 2006 from plasma of pooled US blood donors. Test materials were stored at 2-4°C. On day 0, the test solutions were prepared by diluting the test materials in PBS to the desired concentration (Table 3). The antibody titer in each preparation is given in Table 4.

**Data evaluation:** Survival data were analyzed using the Wilcoxon log rank survival analysis (JMP statistical software, version 4.0, SAS institute). Survival rates in different groups were compared by two-tailed Fisher's exact test. Average days for death were compared by two-tailed Student's T-test.

### Example 1: Monitoring the Presence of WNV specific antibodies in IVIG produced from pooled Israeli-donor plasma.

West Nile Virus has been endemic in Israel for many years. During the design of this study we hypothesized that Israeli plasma and the resulting IVIG preparations contain variable ranges of anti-WNV antibodies. To test our hypothesis, we analyzed batches of immunoglobulin prepared from plasma samples collected from 1998 until 2003 (Fig. 1). All IVIG batches had antibody titers to WNV above a negative control (in this case IVIG manufactured from US plasma; see Table 3).

There are now good indications that after 2002, the Israeli blood donor population was continuously exposed to the virus. According to a report from the Israeli Center for Disease Control (ICDC), after the peak exposure in 2000 (outbreak of West Nile fever, WNF, in Israel), the rate of verified WNF cases was lowered and remained constant during the successive 4 years (Infectious Diseases in Israel, 54 years of surveillance, 1957-2004, ICDC, 2006). However, during 2005 there was a sharp increase in the number of verified cases (at least 5-6 times more than in 2004), and the numbers started to decline during 2006 (ICDC and Central Virology Laboratory, Israeli Ministry of Health, personal communication). The continuous activity of the disease in Israel is a good indicator of exposure of the general population including the blood donor population. This was verified by using our validated ELISA assay to test the presence of WNV antibodies in selected IVIG batches produced from plasma of Israeli donors during 2004-2006. The results showed that the level of WNV antibodies in IVIG preparations produced from Israeli donors is variable; thus, different levels of WNV antibodies are measured in different years and even within the same year (Fig. 2).

### Example 2: Screening of plasma units.

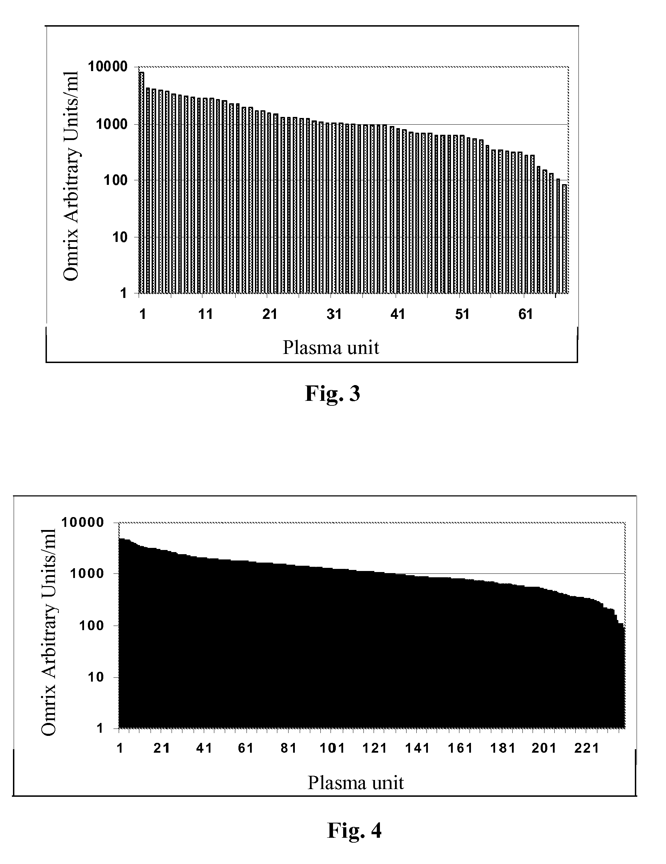

In a preliminary study that was carried out in 2002, a total of 506 samples from Israeli donor plasma units were screened and 67 were found positive for WNV antibodies (about 13% positives) using West Nile Virus, strain NY 385-99. The specific antibody titers within the positives ranged between 85 and 8041 AU/ml. Fig. 3 shows the distribution of titer within the positive samples. The average titer of the positives was 1434 AU/ml, median titer, 971 AU/ml.

In a second study carried out during 2006, we screened a total of about 3000 plasma units. Of these, 238 were found positive for WNV antibodies (about 8% positives). This rate is significantly lower than the rate found in 2002 (p=0.0001 by chi square). The specific antibody titers within the positives ranged between 91 and 4810 AU/ml. The distribution of the positive samples is shown in Fig. 4. The average titer among the positive samples was 1336 AU/ml, median titer, 1091 AU/ml.

### Example 3: Production and specification of high-titer anti-WNV IVIG.

A high titer WNV-IVIG composition (WNIG) was produced by screening plasma units of Israeli donors which is endemic to the virus, and isolating and pooling plasma of donors above 100 AU/ml for antibody specific to the NY-99 strain using ELISA. A total of 267 units (~50 liters) of hyper immune plasma were available for the production of a pilot WNIG batch. The estimated titer of WNV antibodies in the plasma pool comprising the individual units was 904 AU/ml.

A total of 107 gr of IgG were produced (107 vials of 20 ml 5% IVIG solution). The characteristics of the WNIG pilot batch are summarized in Table 1 below.

**Table 1: COA* data of WNIG pilot batch.**

| ***Parameters*** | **Limits**** | **Results** |
|---|---|---|
| Identification | Clear or slightly opalescent and colorless or pale yellow solution. | Complies |
| Ouchterlony | Gives a positive reaction with human protein anti-sera and a negative reaction with animal protein anti-sera. | Complies |
| IgG by Cellulose Acetate electrophoresis | ≥95% | > 97 |
| Anti WNV antibodies by Elisa | | 7302 |
| IgG Content | 42.8 mg/ml - 55.0 mg/ml | 43.3 |
| Anti-Hepatitis A | >2.0IU(IU/ml) | 17 |
| Anti-HBsAg | >0.5 IU/g IgG | 36.3 |
| Anti B19 Abs | >40IU/ml | 127 |
| Diphtheria antitoxin (neutralizing) | >0.6 U of US Standard antitoxin/ml | 1.9 |
| Anti Poliomyelitis 1Abs | >0.1 times the NIH reference lot #176 | 0.55 |
| Anti Measles Abs (Neutralizing) | >0.2 times the NIH reference lot #176 | 0.27 |
| WNV PRNT50 (Neutralizations activity) | For information (WNV/ml) | 4516 |
| Total Protein | 4.5% - 5.5% (w/v) | 5.0 |
| Distribution of molecular size | Monomer and dimer ≥95.00% | 99.61 |
| (HPLC) | Polymers and aggregates ≤3.00% | 0.39 |
| Anticomplementary activity | ≤1 CH₅₀/mg IgG | 0.5 |
| Prekallicrein activator | ≤35 IU/ml | <4.25 |
| Anti-A & Anti-B haemagglutinins | The 1:64 dilutions do not show Agglutination | Complies |
| Tri-n Butyl Phosphate (GC) | ≤5.0 µg/ml (ppm) | 0.2 |
| Triton X-100 (HPLC) | ≤5.0 µg/ml (ppm) | <5.0 |
| IgA content | ≤0.15 mg/ml | 0.07 |
| IgM content | ≤0.1 g/L (≤10 mg/dl) | 0.03 |
| Anti D antibodies | <0.0475IU/ml | <0.024 |
| pH | 5.1 - 6.0 | 5.6 |
| Osmolality | ≥240 mOsmol/kg H₂O | 330 |
| Sodium (Flame photometer) | <20 mmol/l | <1 |
| Maltose | 90-110 g/l | 97 |
| Pyrogens (10 ml/kg Rabbit) | Non Pyrogenic | Non-Pyrogenic |
| Sterility (Steritest) General safety Test | Sterile Complies | Sterile Complies |

| | | |
|---|---|---|
| * COA- certificate of analysis. ** Regular Israeli IVIG from unselected donors. | | |

### Example 4: Preclinical analysis of WNIG in a model of WNV infection.

Several animal models for WNV infection are used for assessment of protocols for prevention and treatment of WNF. A prerequisite for animal model is the establishment of encephalitis phase during the course of disease. The mouse lethal model is considered a very good model and is routinely used for evaluation of vaccines intended to prevent and treat WNV infection. In this model, the challenge virus is given intraperitoneally (IP) to 4-5 weeks old mice. A short viremic phase thus develops (peak at 2 days) followed by infection of the central nervous system (CNS) that starts on day 3-4. The animals usually start showing neurological symptoms towards the end of the first week. The common symptoms include piloerection, partial or complete paralysis and finally death. Usually most of the deaths occur between days 6-10 and very rarely, deaths occur after the second week following infection. In a previous study using this model, it was shown that regular IVIG produced from plasma of Israeli donors was able to protect mice from a lethal dose of WNV (Ben-Nathan, Lustig et al. 2003). The virus strain used in this previous study originated from an Israeli strain isolated in 1952. The present experiments were designed to show the efficacy of WNIG in the mouse model and compare the efficacy of WNIG to that of regular IVIGs produced from plasma of Israeli or US donors. In these experiments an Israeli WNV challenge strain was replaced with a NY99 strain isolated from the target geographic area in the USA.

### Example 5: Virus challenge dose (LD50).

The virus dose was verified by analyzing the vial of stock solution used for preparing the challenge doses. Fig. 5 shows the survival of animals included in the back titration analysis. A clear dose response was achieved; with the 5 PFU dose giving 50% death in the relevant group thus was considered as 1 X LD50.

These results were verified in another set of experiments. Four-five weeks old BALB/c mice were given IP inoculation of 2, 5, 10, 20, 50, 100 or 1000 PFU/mouse. Mortality was monitored daily for 21 days. The combined results of 5 experiments are summarized in Table 2 below. In order to calculate LD₅₀, survival data of 2-20 PFU inoculated mice were plotted against the relevant inoculums (Fig. 6) and a logarithmic trend line was calculated (using EXCEL software). According to the results, the predicted LD₅₀ is 8.3 PFU. For convenience, 5 PFU was considered as the actual LD₅₀ throughout the study.

**Table 2: Survival rate of WNV-infected mice.**

| | Experiment | | | | |
|---|---|---|---|---|---|
| Virus dose (PFU) | #1 | #2 | #3 | #4 | #5 |
| 2 | 7/8 | 8/8 | | | |
| 5 | 3/6 | 5/8 | 4/6 | 4/6 | 7/8 |
| 10 | | | 3/6 | 3/6 | |
| 20 | 1/8 | | | | |
| 50 | | 1/6 | | | 2/8 |
| 100 | 0/8 | 0/8 | 1/6 | 0/8 | 0/8 |
| 1000 | | | | | 0/8 |

### Example 6: Test articles titer.

Three IVIG preparations were used: high titer WNV-IVIG (WNIG), plasma of pooled Israeli blood donors (IVIG-IS), and plasma of pooled US blood donors (IVIG-US). The specific WNV antibody titer in each preparation was analyzed by ELISA and by PRNT50. The results demonstrate that, the fold of enrichment of High titer WNV-IVIG (WNIG) compared to IVIG-IS was about 11 and 10 fold using the validated ELISA and PRNT50 assays, respectively (Table 3).

**Table 3. Anti-WNV antibodies in samples used in the mouse lethal model.**

| Batch # (IVIG preparation) | ELISA | | PRNT50 | |
|---|---|---|---|---|
| | AU/ml | AU/mg | WNU/ml | WNU/mg |
| K44G511 (WNIG) | 7608 | 152 | 9900 | 198 |
| H23131 (IVIG-IS) | 668 | 13 | 1032 | 21 |
| K30G370 (IVIG-US) | 179 | 4 | Not Done | |

### Example 7: Protection of mice from virus challenge.

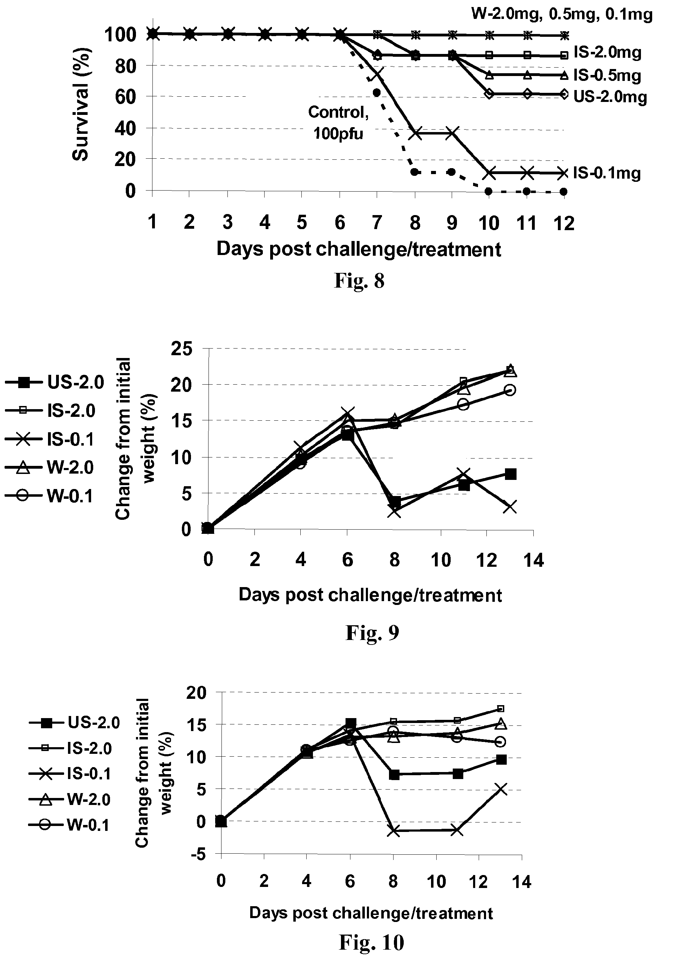

This example illustrates the efficacy of WNIG compared to IVIG-IS and IVIG-US preparations. For this purpose, mice were infected IP with 100 and 20 PFU (20 and 4 X LD50, respectively) of WNV-NY99. Four hours later each mouse received a single injection of 2, 0.5 or 0.1 mg/mouse of WNIG, IVIG-IS (equivalent to 120, 30 and 6 mg/kg respectively) or 2 mg/mouse IVIG-US. Mortality was monitored for 12 days. Figs 7 and 8 show the survival of animals receiving 20 PFU (4 X LD50) and 100 PFU (20 X LD50), respectively. The results indicate that US-source IVIG exhibited a moderate protection against both 20 and 100 PFU even when given at the high dose of 2.0 mg/mouse, equivalent to 120 mg/kg. In comparison, WNIG achieved complete protection with as low as 0.1 mg/mouse, equivalent to 6 mg/kg.

Regular IVIG from Israeli donors gave relatively good protection ranging between 75-87% with 0.5 mg/mouse, equivalent to 30 mg/kg, but had only 12% protection at the lowest dose of 0.1 mg/mouse. Additionally, the results show that 2 mg/mouse IVIG-IS had similar protective efficiency as 0.1 mg/mouse WNIG, i.e., WNIG is about 20 times more potent than IVIG-IS. This 20 times increase in potency is surprising and synergistic in light of the fact that the level of antibodies in the preparations has increased only by 10 or 11 times (see Table 3).

Analysis of the mean weight of the surviving animals during the study was used as a parameter for evaluating the protective efficacy of the IVIG preparations. In each time point the weight of each surviving animal was compared to the initial weight and an average difference was calculated for each treatment group. The results revealed a very similar picture (Figs 9, 10). All of the animals gained about 15% of their initial weight during the first 6 days after infection. In the most affected groups, i.e., US-2.0 mg/mouse and IS-0.1 mg/mouse, the animals started losing weight after day 7, probably reflecting active brain infection that affected the surviving animals.

### Example 8: Protection against lethal WNV infection by human WNIG.

In order to corroborate the results above, another set of experiments was carried out with a prolonged observation period of 21 days. The results were compatible with the previous experiment (Table 4). Treatment with 0.1 mg/mouse of WNIG was sufficient to confer 100% protection against both 20 and 100 PFU of the virus.

The results also show that 0.1 mg/mouse WNIG had similar protective efficiency as 2 mg/mouse IVIG-IS and 0.5 mg/mouse IVIG-IS (100, 93 and 87% survival, respectively), indicating that WNIG of the invention is 5 to 20 more efficient than IVIG-IS against WNV.

IVIG-US showed low efficacy with only 62 and 50% survival for 20 and 100 PFU, respectively, when administered at a high concentration of 2.0 mg/mouse. The above results demonstrate that WNIG is at least 20 times more potent than IVIG from US donors.

Surprisingly, 390 and 912 Arbitrary Units (AU)/kg WNV antibodies originated in Israeli plasma (IVIG-IS and WNIG, respectively) lead to higher survival rate for both 20 and 100 PFU than 480 AU/kg originated in US plasma. These results indicate the qualitative differences between the specific antibodies in US and Israeli donors.

**Table 4: Protective efficacy of WNIG, IVIG-IS and IVIG-US in West Nile virus infected mice.**

| | | | Survival % (Live /Treated) | | |
|---|---|---|---|---|---|
| Treatment (mg/mouse) | Approx. dose per kg^{a} | Ab Titer^{b} | 100 PFU | 20 PFU | 4-20 LD₅₀ |
| No treatment | | 0 | 0 (0/8) | 12 (1/8) | 6 (1/16) |
| WNIG0.1 | 6 mg/kg | 912 | 100 (8/8) | 100 (8/8) | 100 (16/16)^{c} |
| IVIG-IS 0.1 | | 78 | 12 (1/8) | 12 (1/8) | 12 (2/16) |
| WNIG 0.5 | 30 mg/kg | 4,560 | 100 (8/8) | 100 (8/8) | 100 (16/16) |
| IVIG-IS 0.5 | | 390 | 86 (6/7) | 88 (7/8) | 87 (13/15) |
| WNIG 2.0 | 120 mg/kg | 18,240 | 100 (7/7) | 100 (8/8) | 100 (15/15) |
| IVIG-IS 2.0 | | 1,560 | 88 (7/8) | 100 (8/8) | 93 (15/16) |
| IVIG-US 2.0 | | 480 | 62 (5/8) | 50 (4/8) | 56 (9/16) |

| | | | | | |
|---|---|---|---|---|---|
| ^{a} Calculated for 17 gr. animals. ^{b} Arbitrary units of WNV antibodies per kg (measured by ELISA) calculated according to the values in Table 4. ^{c} p<0.001 compared to IVIG-IL 0.1; p=0.007 compared to IVIG-US 2.0. | | | | | |

### Example 9: Dose dependent protection of WNIG.

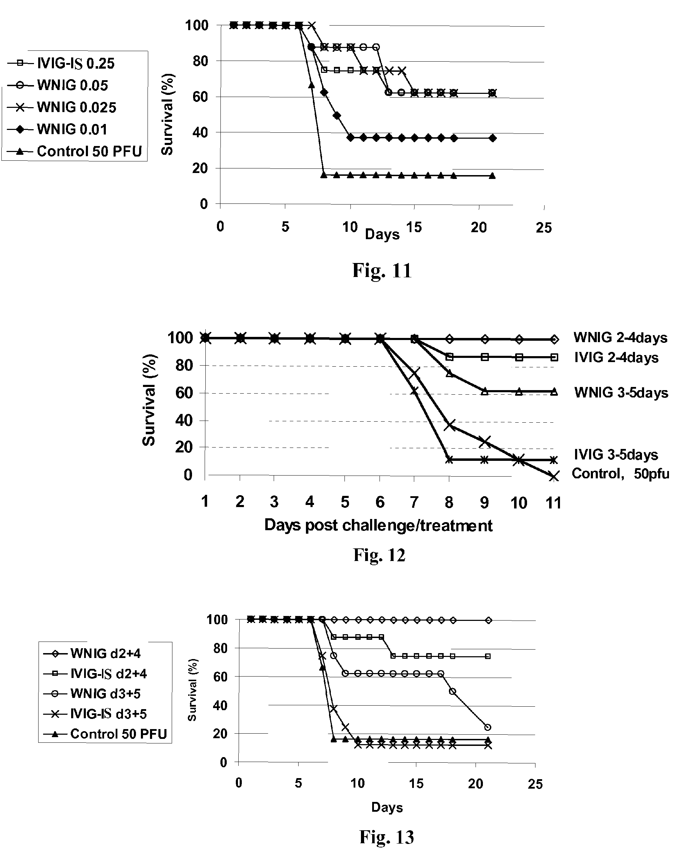

To investigate the therapeutic efficacy of WNIG at amounts which are lower than 0.1 mg/mouse, groups of 5-week old BALB/c mice were infected with 10 X LD50 (50 PFU) of WNV and treated 4 hours later with a single injection of 0.05, 0.025 or 0.01 mg/mouse of WNIG or 0.25 mg/mouse IVIG-IS. Mortality was monitored in mice for a period of 21 days. The control group was given a virus challenge without any treatment (Fig. 11).

The results obtained indicate that all amounts of WNIG tested (0.01, 0.025 and 0.05 mg/mouse) reduced or delayed mortality compared to the control infected mice.

In addition, as shown in the previous experiments, WNIG is 5-10 fold more potent as compared to IVIG-IS since treatment with 0.025 and 0.05 mg/mouse WNIG and 0.25 mg/mouse IVIG-IS lead to similar survival level.

In the untreated WNV-NY99 infected controls, all deaths occurred until day 8 while in the treated mice (0.025 and 0.05 mg of WNIG or 0.25 mg of IVIG-IS) mortality occurred between days 8-15, with a similar delayed mortality pattern.

### Example 10: Efficacy of WNIG treatment in WNV challenged mice administered with WNIG 2 or 3 days after challenge.

Animals were challenged with 50 PFU of virus and were treated with IVIG-IS or WNIG as indicated in Fig. 12. Four groups of animals received two doses of 2 mg/mouse each corresponds to about 120 mg/kg. The first dose was given 2 or 3 days after challenge, and a second dose was given 2 days after the first one. A control group was given a virus challenge without treatment. The animals were monitored for mortality for a period of 11 days. The treatment timing was chosen since in earlier studies it was shown that the virus is present in the brains of the animals 3 days after challenge. A dose of 2 mg/mouse was chosen since this amount conferred a 100 % protection in both IVIG-IS and WNIG 4 hours after challenge. When treatment started 2 days after challenge, both IVIG-IS and WNIG were similarly efficient since the virus was not yet infecting the brain. Three days after challenge, the superiority of WNIG was clearly demonstrated by protecting 5 of the 8 animals while in the IVIG-IS group, only one was spared.

These results were corroborated in another set of experiments on groups of 5-week old BALB/c mice, which was carried out as described above with a prolonged observation period of 21 days (Fig. 13).

The results show that treatment with IVIG-IS and WNIG 2 days following WNV challenge lead to a similar extent of protection for a period of 12 days. However, on the 13^{th} day a decrease in the survival rate was observed in the IVIG-IS-treated animals whereas WNIG conferred a 100% protection for the whole observation period.

In addition, the results indicate that treatment with WNIG on days 3 and 5 after WNV challenge resulted in enhanced survival for the duration of 18 days. Afterwards, a reduction in the survival rate was observed (to about 25%). In comparison, treatment 3 and 5 days after infection with IVIG-IS showed no protection at all and showed about 15% survival rate (lower than the control group). According to these results, the superiority of treatment with WNIG compared to IVIG-IS on days 3 and 5 post infection is demonstrated by postponing the average death day (data not shown) and by conferring a higher survival rate compared to IVIG-IS and control groups. These results indicate the advantage of using WNIG during or prior to active encephalitis.

### Example 11: Efficacy of WNIG at High Doses in WNV Challenged Mice Administered with a Single or a Triple Dose of WNIG.

In order to illustrate the effect of WNIG when given at high doses during the encephalitis phase, BALB/c mice were infected with 10 X LD50 (50 PFU) WNV and treated with a single or triple dose of 8 mg/mouse (corresponds to 470 mg/kg) on day 3, 4, or 5 post challenge (see Table 5). The control group was given a virus challenge without treatment. And a positive control group was treated with 2 mg/mouse WNIG four hours after challenge. The animals were monitored for mortality for a period of 21 days.

According to the results, on day 3 both single and triple injections of high dose of WNIG (8 mg/mouse) resulted in protection of 100% as compared to the previous example wherein a lower dose of WNIG (2 mg/mouse) on day 3 resulted in a final protection of about 25%. When treatment was given 4 days after infection, survival of 69% was achieved compared to 25% of the control group. In all treatment groups a single dose was similar to three doses administration.

**Table 5. Survival rate of WNV infected mice after treatment with 8 mg/mouse WNIG.**

| | | | Survival % (Live /Treated) | | |
|---|---|---|---|---|---|
| Treatment | Challenge | Treatment dose (mg/mouse) | Single dose | Three doses | Combined |
| No treatment | 50 PFU | | | | 25 (2/8) |
| Day 0 | | 2 | | | 100 (8/8) |
| Day 3 or 3, 5, 10 | | 8 or 3 X 8 | 100 (8/8) | 100 (8/8) | 100 (16/16) |
| Day 4 or 4, 6, 10 | | | 63 (5/8) | 75 (6/8) | 69 (11/16) |

### Example 12: WNIG Therapy of immunosuppressed mice challenged with WNV

Adult mice are relatively resistant to WNV but this resistance can be abolished by treatment with Glucocorticoids such as Dexamethasone.

In order to demonstrate the efficiency of WNV antibody under severe immunosuppression conditions, 7 to 8 weeks old mice were administered with subcutaneous injection of 60 µg/mouse Dexamethasone, 2h before and 1 day after infection with 5 or 10 PFU WNV. Mortality was monitored for a period of 21 days. Indeed, the results demonstrated that Dexamethasone administration decreased the survival rate from 58% to 19%. However, IP treatment with 0.2 and 1 mg/mouse WNIG four hours after virus infection led to 100% survival (Table 6). This experiment demonstrates the potential use of WNV antibody therapy under severe immunosuppression.

**Table 6: The effect of WNIG on WNV infection in mice treated with Dexamethasone.**

| Treatment | Survival % (Live /Treated) | | |
|---|---|---|---|
| | 5 PFU | 110 PFU | 15-10 PFU |
| WNV control | 66 (4/6) | 50 (3/6) | 58 (7/12) |
| WNV + Dexamethasone | 13 (1/8) | 25 (2/8) | 19 (3/16)^{a} |
| WNV + Dexamethasone + 1mg WNIG | 100 (8/8) | 100 (8/8) | 100 (16/16) |
| WNV + Dexamethasone + 0.2mg WNIG | 100 (8/8) | 100 (8/8) | 100 (16/16) |

| | | | |
|---|---|---|---|
| * No mortality was observed in a group of 8 control Dexamethasone treated mice (results not shown). ^{a} p=0.004 compared to WNV control; p<0.001 compared to WNIG 0.2 and 1.0 mg. | | | |

### Embodiments of the Invention

1. A method for the preparation of an immunoglobulin composition comprising a high level of neutralizing antibody specific for a pathogenic virus causing a new emerging disease, disorder or condition in a target geographic area, the method comprising: screening blood or blood fractions of a plurality of donors from a population of a geographic area endemic to the virus, in which population the percentage of seropositive donors to said virus is not more than 25%; and isolating and pooling all blood or blood fractions of donors that are positive for antibody specific to the pathogenic virus strain.
2. The method according to embodiment 1, wherein, in said population, the percentage of seropositive donors to said virus is in the range of 1-15%.
3. The method according to embodiment 2, wherein, in said population, the percentage of seropositive donors to said virus is in the range of 8-13%.
4. The method according to any one of embodiments 1 to 3, wherein the screening of blood or blood fractions is carried out with a pathogenic virus strain isolated from a geographic area that is different from the geographic area of the population of donors.
5. The method according to embodiment 4, wherein the screening of blood or blood fractions is carried out with a pathogenic virus strain isolated from or nearby the target geographic area.
6. The method according to any one of embodiments 1 to 5, wherein the method further comprises processing the pooled blood or blood fractions to an intravenous immunoglobulin composition.
7. The method according to embodiment 6, further comprising the step of aliquoting the immunoglobulin composition in a low-volume unit dosage form.
8. The method according to any one of embodiments 1 to 7 wherein the virus is West Nile Virus (WNV).
9. The method according to any one of embodiments 1 to 8, wherein the geographic area endemic to the virus is Israel and/or neighboring territories and the target geographic area is in the United States.
10. A method for the preparation of an immunoglobulin composition comprising a high level of anti-West Nile Virus (WNV) neutralizing antibody for treating or preventing a disease, disorder or condition caused by WNV in a target geographic area, the method comprising: screening blood or blood fractions of a plurality of donors from a population of a geographic area endemic to the virus, in which population the percentage of seropositive donors to said virus is not more than 25%; and isolating and pooling all blood or blood fractions of donors that are positive for antibody against WNV.
11. The method according to embodiment 10, wherein, in said population, the percentage of seropositive donors to said virus is in the range of 1-15%.
12. The method according to embodiment 11, wherein, in said population, the percentage of seropositive donors to said virus is in the range of 8-13%.
13. The method according to any one of embodiments 10 to 12, wherein said blood or blood fractions are positive for WNV isolated from the target geographic area.
14. The method according to any one of embodiments 10 to 13, wherein the method further comprises processing the pooled blood or blood fractions to an intravenous immunoglobulin composition.
15. The method according to embodiment 14, wherein the method further comprises the step of aliquoting the immunoglobulin composition in a low- volume unit dosage form.
16. The method according to any one of embodiments 10 to 15, wherein the geographic area endemic to the virus is Israel and/or neighboring territories and the target geographic area is in the United States.
17. A potent immunoglobulin composition comprising high levels of neutralizing antibodies specific for a pathogenic virus causing a new emerging disease, disorder or condition in a target geographic area obtained by the method according to any one of embodiments 1 to 16.
18. A potent immunoglobulin composition comprising high levels of antibodies specific for WNV of a target geographic area, wherein said composition comprises immunoglobulin from pooled blood or blood fractions of donors that are all positives for antibody against WNV, wherein said donors are from a population of a geographical area endemic to WNV, and wherein said composition has a high level of antibody against the WNV of the target geographical area that is more than 5 times, 10 times, 11 times, or about 20 times higher than the level of anti WNV antibody of immunoglobulin from a regular pool of blood or blood fractions of donors of the geographic area endemic to the virus.
19. A vial or pre-filled syringe containing a low- volume unit dosage of the composition according to any one of embodiments 16 or 18.
20. A pharmaceutical composition for the treatment or prevention of a WNV associated disease, disorder or condition in a target geographical area comprising a pharmaceutically acceptable carrier and an immunoglobulin composition according to embodiment 17 or 18.
21. A method for treating or preventing a WNV associated disease, disorder or condition comprising administering to a subject in need a therapeutically effective amount of an immunoglobulin composition according to any one of embodiments 17, 18 and 20.
22. A method for treating or preventing a WNV associated disease, disorder or condition which requires administration of unreachable excessive amount and/or volume of IVIG from a regular pool of donors of the geographic area endemic to the virus, the method comprising administering to a subject in need a therapeutically effective amount of an immunoglobulin composition according to any one of embodiments 17, 18 and 20.
23. An efficient method for screening and selecting blood or blood fractions comprising a high level of neutralizing antibody specific for a pathogenic virus causing a disease, disorder or condition from numerous blood or blood fractions samples comprising the steps of: screening, using an ELISA assay, blood or blood fraction samples obtained from donors of a population from a geographic area endemic to the virus, in which population the percentage of seropositive donors to said virus is not more than 25%; and selecting blood or blood fraction of donors that are positive for antibody specific for said pathogenic virus.
24. The method according to embodiment 23, wherein, in said population, the percentage of seropositive donors to said virus is in the range of 1-15%.
25. The method according to embodiment 23, wherein, in said population, the percentage of seropositive donors to said virus is in the range of 8-13%.
26. The method according to any one of embodiments 23 to 25, wherein the pathogenic virus is WNV.
27. The method according to any one of embodiments 23 to 26, wherein the ELISA assay is carried out in an automated system.
28. A composition for the treatment or prevention of a WNV associated disease, disorder or condition comprising blood or blood fractions obtainable by the screening method according to any one of embodiments 23 to 27.
29. A method for treating or preventing active encephalitis caused by West Nile Virus comprising administering to a subject in need a therapeutically effective amount of a composition according to any one of embodiments 17, 18, 20 and 28.

### References:

Ben-Nathan, D., S. Lustig, et al. (2003). "Prophylactic and therapeutic efficacy of human intravenous immunoglobulin in treating West Nile virus infection in mice." J Infect Dis 188(1): 5-12.
CDC-USA (2008). "Statistics, Surveillance, and Control of WNV in the USA, http://www.cdc.gov/ncidod/dvbid/westnile/surv&control.htm."
Current Protocols in Immunology, Copyright © 2007 by John Wiley and Sons, Inc. Last updated: 22 Feb 2008 Chapter 2.1
Dobkin, MB and RE Louie. (1986). "High titer cytomegalovirus immune serum globulin." U.S. Patent 4,617,379.
Hamdan, A., P. Green, et al. (2002). "Possible benefit of intravenous immunoglobulin therapy in a lung transplant recipient with West Nile virus encephalitis." Transpl Infect Dis 4(3):160-2.
Hayes, E. B. and D. J. Gubler (2006). "West Nile virus: epidemiology and clinical features of an emerging epidemic in the United States." Annu Rev Med 57: 181-94.
Kondo K, J. Xu, and ES Mocarski (1996). "Human cytomegalovirus latent gene expression in granulocyte-macrophage progenitors in culture and in seropositive individuals." Proc Natl Acad Sci U S A. 93(20):11137-42.
Nash, D., F. Mostashari, et al. (2001). "The outbreak of West Nile virus infection in the New York City area in 1999." N Engl J Med 344(24): 1807-14.
Nur, I. (1999), "A Method for the Purification of Immunoglobulins." WO99/181
Nur, I. and L. Bar (2002), "Method of the inactivation of viruses by a solvent-detergent combination and nanofiltration." U.S. Patent 6,468,733.
Nur, I. et al. (2007), "Intravenous immunoglobulin composition." U.S. Patent Publication 20070037170.Planitzer, C. B., J. Modrof, et al. (2007). "West Nile virus neutralization by US plasma-derived immunoglobulin products." J Infect Dis 196(3):435-40.
Scherret, J. H., M. Poidinger, et al. (2001). "The relationships between West Nile and Kunjin viruses." Emerg Infect Dis 7(4): 697-705.
Shimoni, Z., M. J. Niven, et al. (2001). "Treatment of West Nile virus encephalitis with intravenous immunoglobulin." Emerg Infect Dis 7(4):759.
Shimoni, Z., et al. (2006). "Pharmaceutical compositions and articles of manufacture useful in reversal of a clinical epiosode of an incurable disease and methods of use thereof." U.S. Patent Publication 20060210574.
Slobedman B, and ES Mocarski. (1999) "Quantitative analysis of latent human cytomegalovirus." J Virol. 73(6):4806-12.
Steele, K. E., M. J. Linn, et al. (2000). "Pathology of fatal West Nile virus infections in native and exotic birds during the 1999 outbreak in New York City, New York." Vet Pathol 37(3): 208-24.
Xiao, S. Y., H. Guzman et al.(2001). "West Nile virus infection in the golden hamster (Mesocricetus auratus): a model for West Nile encephalitis." Emerg Infect Dis 7(4):714-21.
Yakub, I., K. M. Lillibridge, et al. (2005). "Single nucleotide polymorphisms in genes for 2'-5'-oligoadenylate synthetase and RNase L inpatients hospitalized with West Nile virus infection." J Infect Dis 192(10): 1741-8.

## Claims

1. A method for the preparation of an immunoglobulin composition comprising a high level of neutralizing antibody specific for a pathogenic virus causing a new emerging disease, disorder or condition in a target geographic area, the method comprising:
screening blood or blood fractions of a plurality of donors from a population of a geographic area endemic to the virus, in which population the percentage of seropositive donors to said virus is not more than 25%, in the range of 1-15%, or in the range of 8-13%; and
isolating and pooling all blood or blood fractions that are positive for antibody specific to the pathogenic virus strain.

2. The method according to claim 1, wherein the screening of blood or blood fractions is carried out with a pathogenic virus strain isolated from a geographic area that is different from the geographic area of the population of donors.

3. The method according to claim 2, wherein the screening of blood or blood fractions is carried out with a pathogenic virus strain isolated from or nearby the target geographic area.

4. A method for the preparation of an immunoglobulin composition comprising a high level of anti-West Nile Virus (WNV) neutralizing antibody for treating or preventing a disease, disorder or condition caused by WNV in a target geographic area, the method comprising:
screening blood or blood fractions of a plurality of donors from a population of a geographic area endemic to the virus, in which population the percentage of seropositive donors to said virus is not more than 25%, in the range of 1-15%, or in the range of 8-13%; and
isolating and pooling all blood or blood fractions that are positive for antibody against WNV.

5. The method according to claim 4, wherein said blood or blood fractions are positive for WNV isolated from the target geographic area.

6. The method according to any one of claims 1 to 5, wherein the method further comprises processing the pooled blood or blood fractions to an intravenous immunoglobulin composition.

7. The method according to any one of claims 1 to 6, wherein the geographic area endemic to the virus is Israel and/or neighboring territories and the target geographic area is in the United States.

8. A potent immunoglobulin composition comprising high levels of neutralizing antibodies specific for a pathogenic virus causing a new emerging disease, disorder or condition in a target geographic area obtained by the method according to any one of claims 1 to 7.

9. A potent immunoglobulin composition comprising high levels of antibodies specific for WNV of a target geographic area, wherein said composition comprises immunoglobulin from pooled blood or blood fractions of donors that are all positives for antibody against WNV, wherein said donors are from a population of a geographical area endemic to WNV, and wherein said composition has a high level of antibody against the WNV of the target geographical area that is more than 5 times, 10 times, 11 times, or about 20 times higher than the level of anti WNV antibody of immunoglobulin from a regular pool of blood or blood fractions of donors of the geographic area endemic to the virus.

10. A vial or pre-filled syringe containing a low-volume unit dosage of the composition according to claim 8 or 9.

11. A pharmaceutical composition for the treatment or prevention of a WNV associated disease, disorder or condition in a target geographical area comprising a pharmaceutically acceptable carrier and an immunoglobulin composition according to claim 8 or 9.

12. An efficient method for screening and selecting blood or blood fractions comprising a high level of neutralizing antibody specific for a pathogenic virus causing a disease, disorder or condition from numerous blood or blood fractions samples comprising the steps of: screening, using an ELISA assay, blood or blood fraction samples obtained from donors of a population from a geographic area endemic to the virus, in which population the percentage of seropositive donors to said virus is not more than 25%, in the range of 1-15%, or in the range of 8-13%; and selecting blood or blood fraction of donors that are positive for antibody specific for said pathogenic virus.

13. The method according to claim 12, wherein the pathogenic virus is WNV.

14. A composition for the treatment or prevention of a WNV associated disease, disorder or condition comprising blood or blood fractions obtainable by the screening method according to claim 13.

15. Use of a composition according to any one of claims 8, 9, 11 or 14 for treating or preventing a WNV associated disease, disorder or condition such as active encephalitis caused by West Nile Virus and/or a WNV associated disease, disorder or condition which requires unreachable excessive amount and/or volume of IVIG from a regular pool of donors of the geographic area endemic to the virus.
